# EUROPEAN PATENT SPECIFICATION

(11) **EP 2 756 739 B1**
(45) Date of publication and mention of the grant of the patent: **28.11.2018**
(21) Application number: 12832065.2
(22) Date of filing: 14.09.2012
(51) Int. Cl.: H05H 1/46, A61L 2/00

(54) **COLD PLASMA STERILIZATION DEVICES AND ASSOCIATED METHODS**
KALTPLASMASTERILISATIONSVORRICHTUNGEN UND DAMIT ASSOZIIERTE VERFAHREN
APPAREILS DE STÉRILISATION PAR PLASMA FROID ET PROCÉDÉS ASSOCIÉS

(30) Priority: 15.09.2011 US 201161535250 P
(43) Date of publication of application: 23.07.2014
(73) Proprietor: Cold Plasma Medical Technologies, Inc., Scottsdale, AZ 85255 (US)
(72) Inventor: WATSON, Gregory, A., Sanford, FL 32771 (US); JACOFSKY, Marc, C., Phoenix, AZ 85086 (US); JACOFSKY, David, J., Peoria, AZ 85383 (US)
(74) Representative: Round, Edward Mark
(86) International application number: PCT/US2012/055607
(87) International publication number: WO 2013/040481

(56) References cited:
- US-A- 6 096 564
- US-A1- 2002 187 066
- US-A1- 2006 162 741
- US-A1- 2006 244 386
- US-A1- 2008 179 286
- US-B2- 6 474 060
- US-B2- 6 956 329
- US-B2- 7 192 553
- US-B2- 7 633 231

## Description

### BACKGROUND OF THE INVENTION

### Field of the Art

The present invention relates to devices and methods for creating cold plasmas, and, more particularly, to cold plasma sterilization methods and application devices.

### Background Art

Atmospheric pressure hot plasmas are known to exist in nature. For example, lightning is an example of a DC arc (hot) plasma. Many DC arc plasma applications have been achieved in various manufacturing processes, for example, for use in forming surface coatings. Atmospheric pressure cold plasma processes are also known in the art. Most of the at or near atmospheric pressure cold plasma processes are known to utilize positive to negative electrodes in different configurations, which release free electrons in a noble gas medium.

Devices that use a positive to negative electrode configuration to form a cold plasma from noble gases (helium, argon, etc.) have frequently exhibited electrode degradation and overheating difficulties through continuous device operation. The process conditions for enabling a dense cold plasma electron population without electrode degradation and/or overheating are difficult and challenging to achieve.

In another challenging area, autoclaves continue to be used for sterilization of hospital equipment, particularly surgical instruments. However, the use of autoclaves poses several disadvantages that include the following. First, the time needed to cycle an autoclave system (e.g., up to 45 minutes for a full cycle) is substantial, and includes the need for significant cool down time. Second, the repeated temperature swings are rough on equipment, and the use of steam weathers metals. Third, autoclaves are large pieces of equipment with high upkeep costs and frequent downtime. Finally, when a surgical instrument is dropped or otherwise contaminated in an operating room, it must be "flashed" in the autoclave. This is a short cycle (e.g. 15-20 minutes) of high heat and pressure. These surgical instruments come back to the operating room very hot and therefore must cool prior to their use. During this time period, the patient is under anesthetic and likely has an opened wound, with resulting increased potential complications. It is therefore desirable to have an improved method of rapidly sterilizing surgical instruments without the undesirable heating effects, exposure to steam and length time periods associated with autoclaves.

US 2006/0244386 discloses a dielectric barrier plasma discharge device having a pair of electrodes spaced apart by an electrode gap.

US 2008/179286 discloses a dielectric barrier discharge plasma generator including a dielectric chamber.

US 2006/0162741 discloses a method and apparatus for cleaning and surface conditioning objects using plasma.

US 2002/0187066 discloses an apparatus for sterilizing an article using capillary discharge atmospheric pressure plasma.

### BRIEF SUMMARY OF THE INVENTION

As noted above, autoclaves have a number of disadvantages in their use in the sterilization of medical equipment. It is therefore desirable to have an improved method of rapidly sterilizing surgical instruments without the undesirable heating effects and exposure to steam.

Non-thermal gas plasmas (i.e., cold plasmas) have been shown to be effective at the destruction of many pathogens. In addition to their usefulness in the destruction of pathogens, it is also desirable to recirculate the gas used for cold plasma generation. Recirculation not only increases the efficiency of a cold plasma system, but also reduces the operating costs of such a system. In order to achieve effective sterilization of surfaces, and more specifically surgical instruments, contact times of several minutes may be necessary. To effect longer contact times, it is desirable to have a chamber that can contain one or more instruments and a volume of plasma. This description embodies the concept, in device and technique, for creating a plasma sterilization chamber and recirculating the feed/source specialty gas which would otherwise be lost to ambient air conditions. The contained CP recirculation unit shows how a noble gas can be used repeatedly in a cold plasma reaction chamber by way of electron separation in the reaction chamber, and electron attraction back to the normal atomic orbit in the non-energized part of the recirculation unit. This system works at or near atmospheric pressure levels, requiring no substantial additional pressure or vacuum.

An embodiment of a cold plasma sterilization device is provided according to claim 1

Another embodiment is provided according to claim 7 regarding a method of generating a cold plasma for sterilisation.

### BRIEF DESCRIPTION OF THE DRAWINGS/FIGURES

FIGs. 1A and 1B are cutaway views of the hand-held atmospheric harmonic cold plasma device, in accordance with an example useful for understanding the present invention.
FIGs. 2A and 2B illustrate an embodiment of the cold plasma device without magnets, in accordance with an example useful for understanding the present invention.
FIG. 3 is an exemplary circuit diagram of the power supply of a cold plasma device, in accordance with an example useful for understanding the present invention.
FIG. 4 illustrates the generation of cold plasma resulting using a dielectric barrier discharge principle, in accordance with embodiments of the present invention.
FIG. 5 illustrates a cold plasma sterilization device, in accordance with an embodiment of the present invention.
FIG. 6 illustrates a recirculating cold plasma sterilization device, in accordance with an embodiment of the present invention.
FIG. 7 illustrates a plasma chamber of a recirculating cold plasma sterilization device, in accordance with an embodiment of the present invention.
FIG. 8 illustrates the cold plasma emanating from a cold plasma sterilization device, in accordance with an embodiment of the present invention.
FIG. 9 illustrates the cold plasma emanating from a cold plasma sterilization device, in accordance with an embodiment of the present invention.
FIG. 10 illustrates a method of sterilizing an object using a cold plasma sterilization device, in accordance with an embodiment of the present invention.
FIG. 11 illustrates a method of sterilizing an object using a cold plasma sterilization device that uses plasma chamber purging, in accordance with an embodiment of the present invention.

### DETAILED DESCRIPTION OF THE INVENTION

Cold temperature atmospheric pressure plasmas have attracted a great deal of enthusiasm and interest by virtue of their provision of plasmas at relatively low gas temperatures. The provision of a plasma at such a temperature is of interest to a variety of applications, including wound healing, anti-bacterial processes, various other medical therapies and sterilization.

### Cold Plasma Application Device

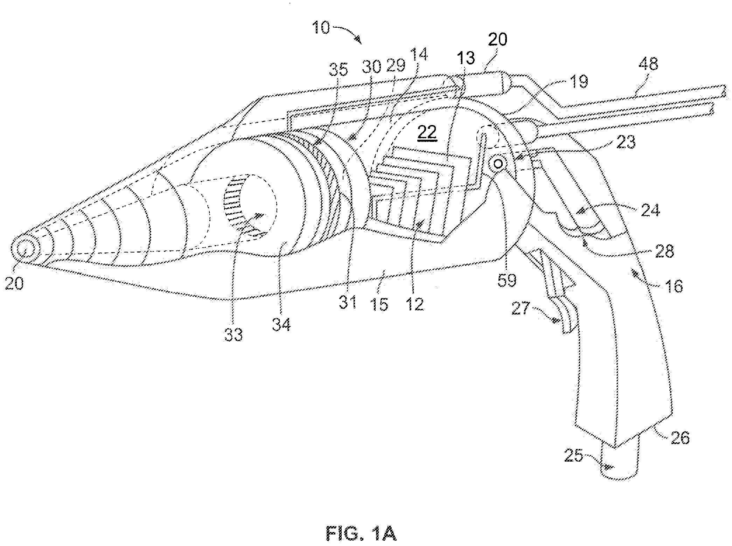

To achieve a cold plasma, a cold plasma device typically takes as input a source of appropriate gas and a source of high voltage electrical energy, and outputs a plasma plume. FIG. 1A illustrates such a cold plasma device. Previous work by the inventors in this research area has been described in U.S. Provisional Patent Application No. 60/913,369, U.S. Non-provisional Application No. 12/038,159 (that has issued as U.S. Patent No. 7,633,231) and the subsequent continuation applications (collectively "the '369 application family"). The following paragraphs discuss further the subject matter from this application family further, as well as additional developments in this field, which are useful for understanding the present invention.

The '369 application family describes a cold plasma device that is supplied with helium gas, connected to a high voltage energy source, and which results in the output of a cold plasma. The temperature of the cold plasma is approximately 65-120 degrees F (preferably 65-99 degrees F), and details of the electrode, induction grid and magnet structures are described. The voltage waveforms in the device are illustrated at a typical operating point in '369 application family.

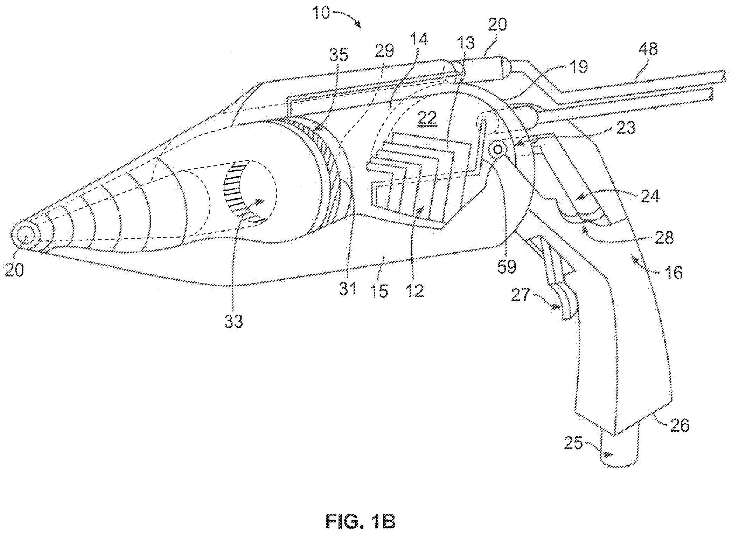

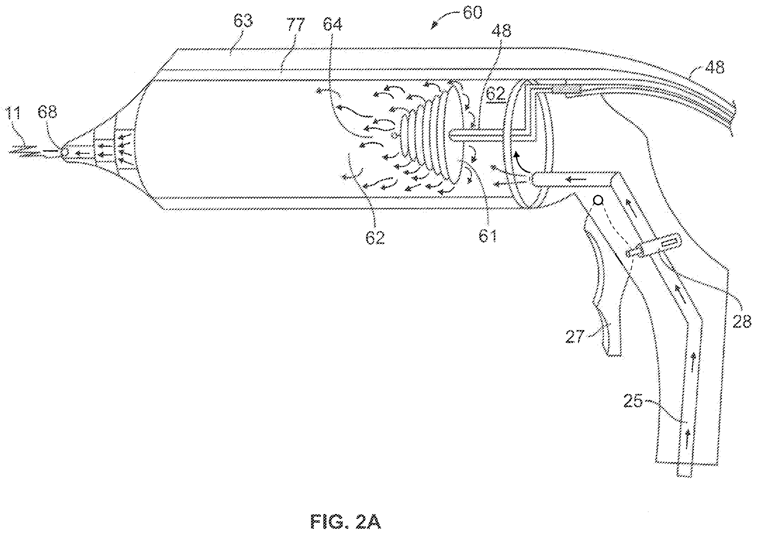

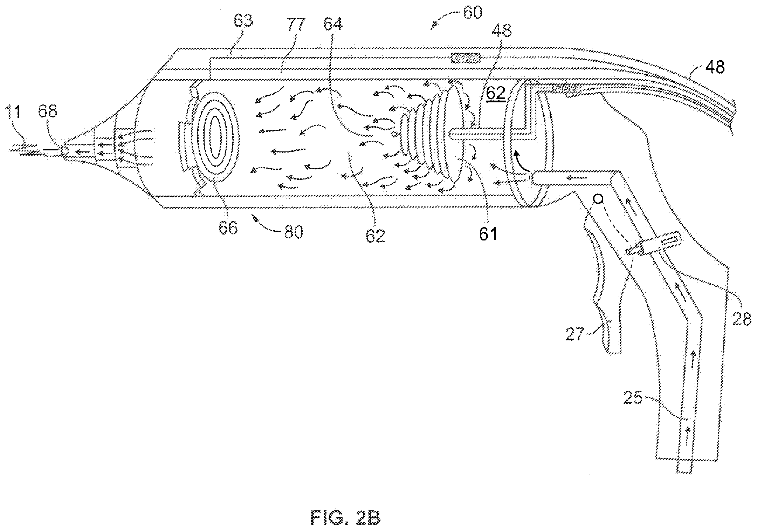

In a further example to that described in the '369 application, useful for understanding the present invention, plasma is generated using an apparatus without magnets, as illustrated in FIGs. 2A and 2B. In this magnet-free environment, the plasma generated by the action of the electrodes 61 is carried with the fluid flow downstream towards the nozzle 68. FIG. 2A illustrates a magnet-free example, useful for understanding the present invention, in which no induction grid is used. FIG. 2B illustrates a magnet-free example in which induction grid 66 is used. FIG. 1B illustrates the same example as illustrated FIG. 2B, but from a different view. Although these examples illustrate the cold plasma is generated from electrode 12, other examples do not power the cold plasma device using electrode 12, but instead power the cold plasma device using induction grid 66.

In both a magnet and a magnet-free example, the inductance grid 66 is optional. When inductance grid 66 is present, it provides ionization energy to the gas as the gas passes by. Thus, although the inductance grid 66 is optional, its presence enriches the resulting plasma.

As noted above, the inductance grid 66 is optional. When absent, the plasma will nevertheless transit the cold plasma device and exit at the nozzle 68, although in this case, there will be no additional ionization energy supplied to the gas as it transits the latter stage of the cold plasma device.

As noted with respect to other examples, magnetic fields can be used in conjunction with the production of cold plasmas. Where present, magnetic fields act, at least at some level, to constrain the plasma and to guide it through the device. In general, electrically charged particles tend to move along magnetic field lines in spiral trajectories. As noted elsewhere, other examples can comprise magnets configured and arranged to produce various magnetic field configurations to suit various design considerations. For example, in one example as described in the previously filed '369 application family, useful for understanding the present invention, a pair of magnets may be configured to give rise to magnetic fields with opposing directions that act to confine the plasma near the inductance grid.

### Cold Plasma Unipolar High Voltage Power Supply

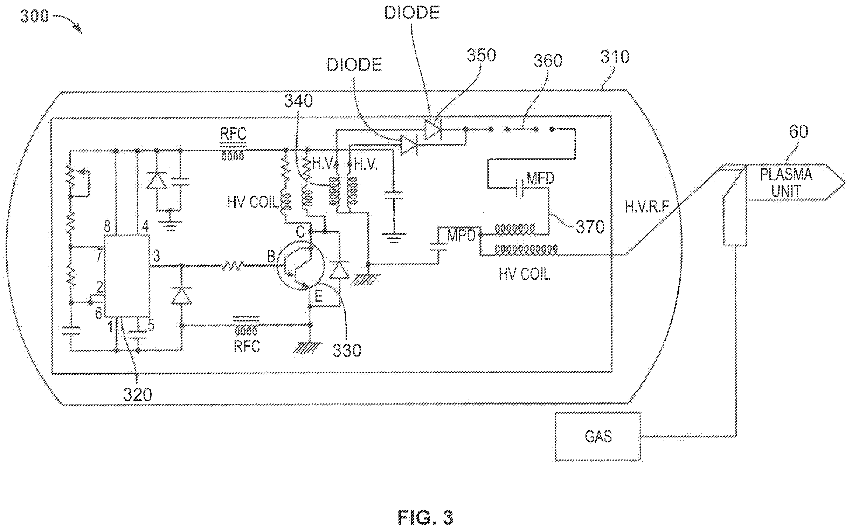

The '369 application family also illustrates an example of the unipolar high voltage power supply architecture and components used therein, useful for understanding the present invention. The circuit architecture is reproduced here as FIG. 3, and this universal power unit provides electrical power for a variety of embodiments described further below. The architecture of this universal power unit includes a low voltage timer, followed by a preamplifier that feeds a lower step-up voltage transformer. The lower step-up voltage transformer in turn feeds a high frequency resonant inductor-capacitor (LC) circuit that is input to an upper step-up voltage transformer. The output of the upper step-up voltage transformer provides the output from the unipolar high voltage power supply.

FIG. 3 also illustrates an example of the unipolar high voltage power supply 310 architecture. In this implementation, a timer integrated circuit such as a 555 timer 320 provides a low voltage pulsed source with a frequency that is tunable over a frequency range centered at approximately 1 kHz. The output of the 555 timer 320 is fed into a preamplifier that is formed from a common emitter bipolar transistor 330 whose load is the primary winding of the lower step-up voltage transformer 340. The collector voltage of the transistor forms the output voltage that is input into the lower step-up voltage transformer. The lower step-up transformer provides a magnification of the voltage to the secondary windings. In turn, the output voltage of the lower step-up voltage transformer is forwarded to a series combination of a high voltage rectifier diode 350, a quenching gap 360 and finally to a series LC resonant circuit 370. As the voltage waveform rises, the rectifier diode conducts, but the quench gap voltage will not have exceeded its breakdown voltage. Accordingly, the quench gap is an open circuit, and therefore the capacitor in the series LC resonant circuit will charge up. Eventually, as the input voltage waveform increases, the voltage across the quench gap exceeds its breakdown voltage, and it arcs over and becomes a short circuit. At this time, the capacitor stops charging and begins to discharge. The energy stored in the capacitor is discharged via the tank circuit formed by the series LC connection.

Continuing to refer to FIG. 3, the inductor also forms the primary winding of the upper step-up voltage transformer 340. Thus, the voltage across the inductor of the LC circuit will resonate at the resonant frequency of the LC circuit 370, and in turn will be further stepped-up at the secondary winding of the upper step-up voltage transformer. The resonant frequency of the LC circuit 370 can be set to in the high kHz - low MHz range. The voltage at the secondary winding of the upper step-up transformer is connected to the output of the power supply unit for delivery to the cold plasma device. The typical output voltage is in the 10 - 150 kV voltage range. Thus, voltage pulses having a frequency in the high kHz - low MHz range can be generated with an adjustable repetition frequency in the 1 kHz range. The output waveform is shaped similar to the acoustic waveform generated by an impulse such as a bell is struck with a hammer. Here, the impulse is provided when the spark gap (or SCR) fires and produces the voltage pulse which causes the resonant circuits in the primary and secondary sides of the transformer to resonate at their specific resonant frequencies. The resonant frequencies of the primary and the secondary windings are different. As a result, the two signals mix and produce the unique 'harmonic' waveform seen in the transformer output. The net result of the unipolar high voltage power supply is the production of a high voltage waveform with a novel "electrical signature," which when combined with a noble gas or other suitable gas, produces a unique harmonic cold plasma that provides advantageous results in wound healing, bacterial removal and other applications.

The quenching gap 360 is a component of the unipolar high voltage power supply 310. It modulates the push/pull of electrical energy between the capacitance banks, with the resulting generation of electrical energy that is rich in harmonic content. The quenching gap can be accomplished in a number of different ways, including a sealed spark gap and an unsealed spark gap. The sealed spark gap is not adjustable, while unsealed spark gaps can be adjustable. A sealed spark gap can be realized using, for example, a DECI-ARC 3000 V gas tube from Reynolds Industries, Inc. Adjustable spark gaps provide the opportunity to adjust the output of the unipolar high voltage power supply and the intensity of the cold plasma device to which it is connected. In a further example that incorporates a sealed (and therefore non-adjustable) spark gap, thereby ensuring a stable plasma intensity.

In an example of the unipolar high voltage power supply, useful for understanding the present invention, a 555 timer 320 is used to provide a pulse repetition frequency of approximately 150-600 Hz. As discussed above, the unipolar high voltage power supply produces a series of spark gap discharge pulses based on the pulse repetition frequency. The spark gap discharge pulses have a very narrow pulse width due to the extremely rapid discharge of capacitive stored energy across the spark gap. Initial assessments of the pulse width of the spark gap discharge pulses indicate that the pulse width is approximately 1 nsec. The spark gap discharge pulse train can be described or modeled as a filtered pulse train. In particular, a simple resistor-inductor-capacitor (RLC) filter can be used to model the capacitor, high voltage coil and series resistance of the unipolar high voltage power supply. In an example, the spark gap discharge pulse train can be modeled as a simple modeled RLC frequency response centered in the range of around 100 MHz. Based on the pulse repetition frequency of 192 Hz, straightforward signal analysis indicates that there would be approximately 2,000,000 individual harmonic components between DC and 400 MHz.

In another example of the unipolar high voltage power supply described above, a 556 timer or any timer circuit can be used in place of the 555 timer 320. In comparison with the 555 timer, the 556 timer provides a wider frequency tuning range that results in greater stability and improved cadence of the unipolar high voltage power supply when used in conjunction with the cold plasma device.

### Cold Plasma Sterilization Device

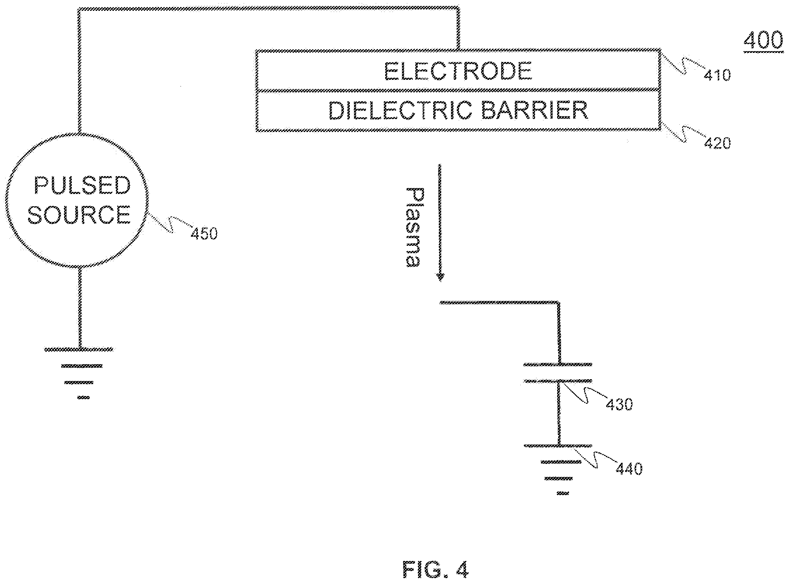

Devices, other than the cold plasma device illustrated above in FIG. 1, can also generate cold plasma. For example, cold plasma can also be generated by a dielectric barrier discharge device, which relies on a different process to generate the cold plasma. As FIG. 4 illustrates, a dielectric barrier discharge (DBD) device 400 contains one metal electrode 410 covered by a dielectric layer 420. The electrical return path 430 is formed by the ground 440 that can be provided by the substrate undergoing the cold plasma treatment. Energy for the dielectric barrier discharge device 400 can be provided by a power supply 450, such as that described above and illustrated in FIG. 3. More generally, energy is input to the dielectric barrier discharge device in the form of pulsed electrical voltage to form the plasma discharge. By virtue of the dielectric layer, the discharge is separated from the metal electrode and electrode etching is reduced. The pulsed electrical voltage can be varied in amplitude and frequency to achieve varying regimes of operation.

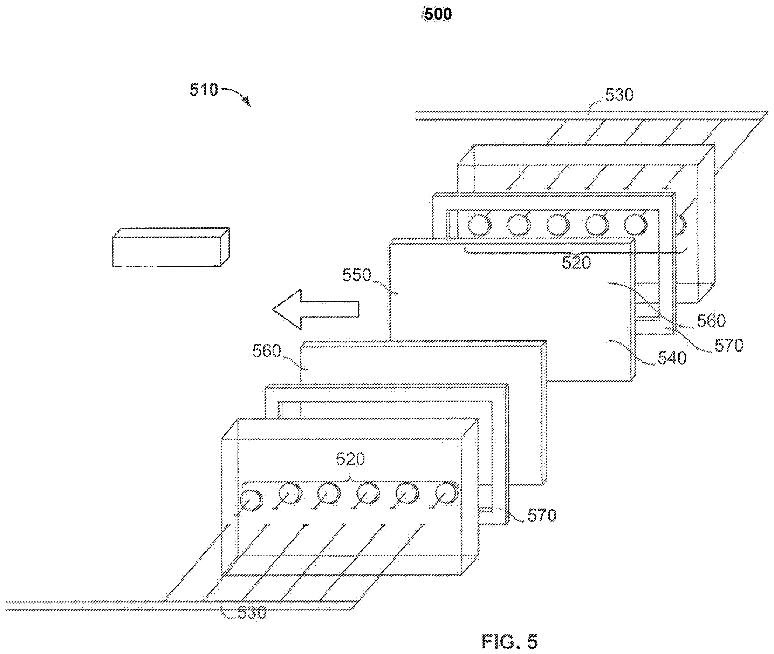

In an exemplary embodiment of the present invention, a sterilization device is provided as shown in FIG. 5. Cold plasma sterilization device 500 has plasma chamber 510 through which gas flow occurs. Gas enters at gas input 540 and exits at gas output 550. Plasma chamber 510 is surrounded by electrodes 520 which are connected to electrical input line 530. Separating plasma chamber 510 from electrodes 520 is a dielectric barrier 560. In order to ensure a gas-tight seal, one or more gaskets 570 can be used. This device has an elongated insulating structure surrounded by a conductor. Plasma chamber 510 can be made of insulating material, including acrylic, plastic, ceramic and the like. Similarly, dielectric barrier 560 can be made of any suitable dielectric material sufficient to withstand the high voltages applied to the electrodes. For example, dielectric barrier 560 can be made of a suitable dielectric material, such as ceramic, polytetrafluoroethylene (PTFE), quartz and the like. Plasma chamber 510 can be any shape including a cylinder. Electrical input line 530 is configured to be connected to a pulsed power supply (not shown). Pulsed power supply provides a source of pulsed electrical source of appropriate voltage and frequency.

As noted above, plasma chamber 510 is a chamber in which gas of an appropriate composition can be presented for gas flow through to an output orifice, such as gas output 550. In a typical example, the gas is helium. Other gases include a helium-oxygen gas combination, although other gases and gas combinations can be used. When electrical energy is applied to device, a cold plasma is formed in the gas. Stray capacitance in plasma chamber 510 will flow to ground to complete the electrical circuit and result in the formation of ionized gas or plasma (albeit somewhat diffuse). A target object (e.g., an object to be sterilized) can be placed in an object holder within plasma chamber 510. If the object holder has a connection, or a suitable capacitance, to ground, such a connection will result in a greater intensity of the plasma. The cold plasma can be visual in that a non-transparent color will become evident upon the provision of energy to the gas. This type of cold plasma device can be used for the sterilization of surgical implants and instruments, where a small size model is suitable for use in operating room, laboratory, medical office, etc., and a large size model is suitable for central sterilization processing in a hospital, medical supply or manufacturing facility.

Gas can be used once and released. Alternatively, the gas can be re-used or recycled (i.e., recirculated). Advantages obtained by recirculating the gas include the following. First, gases, and in particular noble gases such as helium, are expensive. Second, power utilization can be reduced. Fresh gas that enters the system for the first time requires high energy levels to achieve ionization, while returning gas in a recirculation system retains an elevated energy level when it returns to the plasma chamber. Consequently, recirculation allows for potentially a lower power consumption. Third, certain working environments do not permit large volumes of gas (e.g., noble gas) in a contained occupied space such as an operating room due to the risk of potential suffocation. Furthermore, when an ionized noble gas mixes with ambient air, reactive molecules such as ozone are produced, which are potential irritants. Finally, a recirculation process causes turbulence that ensures the cold plasma is well distributed in the treatment chamber, and therefore reaches into the inner lumina of tools (e.g., cannulated drill bits, laparoscopy tools).

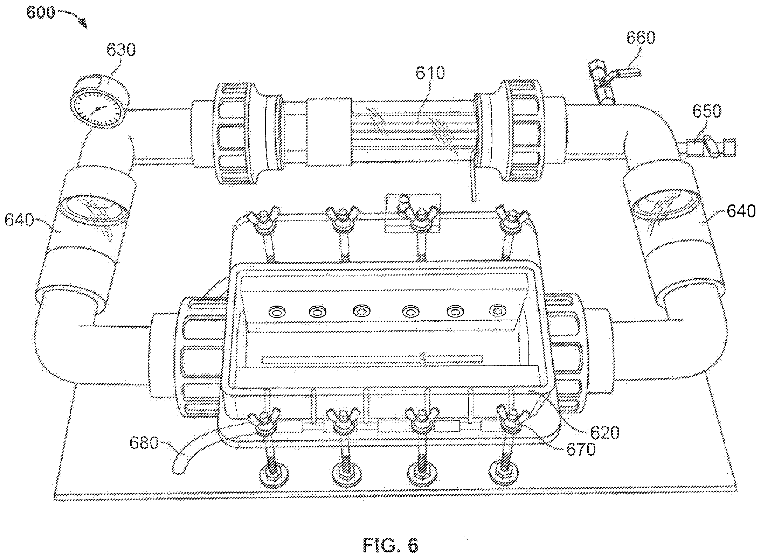

In an exemplary embodiment of the present invention, a low gas consumption embodiment 600 of the cold plasma device is illustrated in FIG. 6. Any appropriate gas can be used, with helium being a possible gas. Gas is input to the recirculatory system via an fill port 650. Gas can be released from the recirculatory system via exit port 660. A recirculate pump 610, such as a circulation fan, forces the gas in a clockwise direction from the fill port 650 to a plasma chamber 620, where the gas encounters one or more dielectric barrier device structures of the type illustrated in FIG. 5. These dielectric barrier device structures are coupled to one or more appropriate high voltage pulsed power sources of the type illustrated in FIG. 3 and described above. Gas exits from plasma chamber 620 to rejoin the recirculation loop in a clockwise fashion. Motion of the gas in a counter-clockwise fashion also falls within the scope of embodiments of the present invention. In the recirculatory system, the plasma-carrying gas can exit through an exit port 660 for possible external application to a treatment area of interest. Recirculate pump 610, such as a circulation fan, can also control the degree of ionization of the resulting plasma, as well as the timing of the sterilization of the treatment area. View tubes 640 can be optionally added to provide the ability to visually examine the gas flow, and presence of the cold plasma. Pressure gauge 630 can be optionally added to provide additional control and measurements of the performance of the cold plasma recirculatory system 600.

A typical use model of the cold plasma sterilization system 600 is described below. An object, such as a medical instrument, to be sterilized is placed into plasma chamber 620. A suitable gas source, such as a noble gas source is connected to the fill port 650 and both the fill port 650 and exit port 660 are opened until plasma chamber 620 contains only noble gas. At this point, fill port 650 and exit port 660 are both closed. The electrical energy is next provided to electrical input 680, which is coupled to electrodes similar to electrodes 520 illustrated in FIG. 5. The electrical energy causes cold plasma to form in plasma chamber 620. Recirculate pump 610, such as a recirculation fan, is turned on. The action of recirculate pump 610 draws some of the ionized gas out of plasma chamber 620 to the left, as illustrated in FIG. 6. Some ionized gas may be visible in the left most view tube 640 shown above. The plasma is returning to its ground state with increasing distance from plasma chamber 620. The reconstituted noble gas passes by recirculate pump 610 (i.e., recirculation fan, or other suitable pumping mechanism), and returns to plasma chamber 620 to be ionized again. This creates a continuous flow of cold plasma through plasma chamber 620 which serves to increase contact between the plasma and object to be sterilized (e.g., medical instrument), particularly in the inner spaces of the to-be-sterilized object, while maintaining cool temperatures. In an embodiment, the recirculate pump 610 can be variable speed in order to provide a variety of operating regimes commensurate with different gas flow speeds.

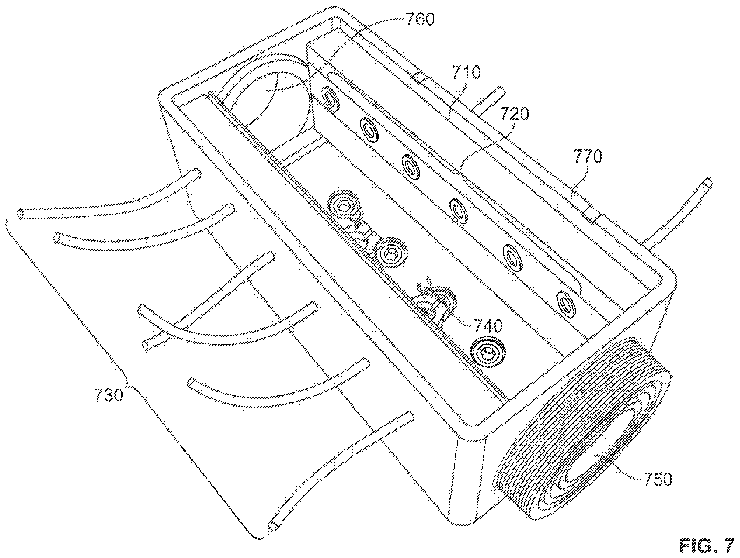

Referring to FIG. 7, which shows further details of plasma chamber 620, there are six electrodes 720 placed on either side of this ionization chamber 710, for a total of twelve electrodes 20. In other examples useful for understanding the invention, electrodes 720 can be completely on one side of plasma chamber 620, or divided into two or more groups of electrodes distributed either randomly inside plasma chamber 620 or in distributed in a non-random fashion inside plasma chamber 620. There are two metallic conductive stands 740 in the center of plasma chamber to receive the object to be sterilized. These metallic conductive stands 740 are coupled to a ground path. In other embodiments, electrodes 720 can be positioned equally about the center of metallic conductive stand 740, or center of metallic conductive stands 740, when multiple metallic conductive stands 740 are in use. When plasma chamber 620 is filled with gas via gas port 750 (gas port 760 would provide the exit port for the gas) and the energy is activated via electrical wires 730, the energy flows through the gas toward the object to-be-sterilized to return to ground. In doing so, the energy ionizes the gas within plasma chamber 620 and bathes the object to-be-sterilized (e.g., medical instrument) in plasma thereby sanitizing its surfaces.

Referring to both FIGs. 6 and 7, in an exemplary embodiment of plasma chamber 620 plasma chamber 620 can be fitted with an acrylic top cover 770 (e.g., seal plate), and a series of fasteners 670 that maintain cover 770 in place during operation. For example, the thickness of the acrylic top cover 770 can be 0.75 (19 mm), with a series of 5/16" x 6" (7.9 mm x 152 mm) bolts used as fasteners. Fasteners 670 (e.g., bolts) are present to allow access to plasma chamber 620, but maintain positive pressure when plasma chamber 620 is being purged of room air and filled with the required gas, e.g., noble gas. A suitable hinge and latch system, studs and nuts or other equivalent mechanisms can be used to implement fasteners 670.

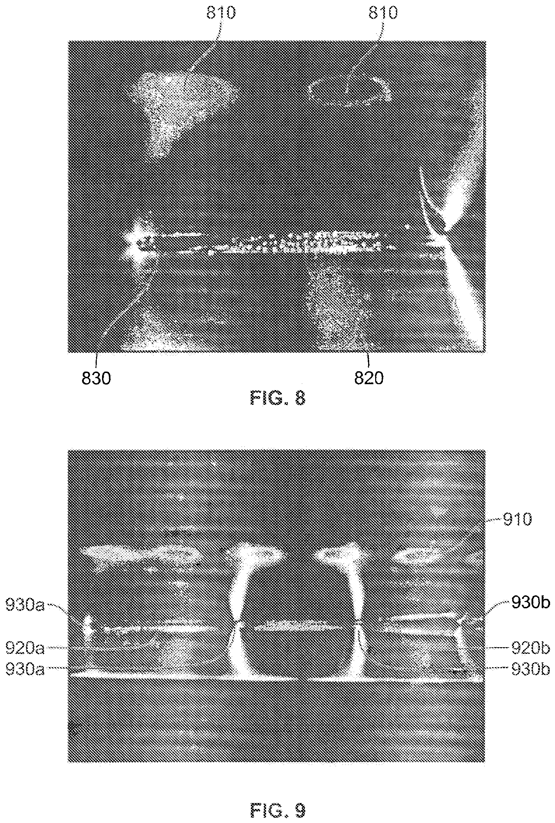

FIG. 8 illustrates the internal environment of plasma chamber showing the distal end of a cannulated acorn reamer 820, the object undergoing sterilization. Two electrodes 810 are visible in the upper portion of the illustration. One of the ground stands 830 holding the cannulated acorn reamer 820 is visible to the right side of FIG. 8. The iridescent balls of light are focal energy flow from the plasma to the cannulated acorn reamer 820, which are likely due to fine scale surface irregularities.

FIG. 9 illustrates a lateral view of the entire cannulated acorn reamer 920 in plasma chamber 620 during system recirculation mode. The six electrodes 910 on one side of plasma chamber 620 are clearly visible. The more intense ionization is noted at the ground stands 930, while a fine plasma covers the whole of plasma chamber 620.
Without the circulating air flow due to, for example, recirculate pump 610, the distribution of the cold plasma would be more coarse and uneven.

In a further embodiment, plasma chambers 620 can be configured in series to allow multiple simultaneous objects to be sterilized in a row of chambers, all connected to the same recirculatory gas system. A bypass port and valve assembly could accompany each chamber so that if one chamber were to be opened, the power is cut and gas is bypassed so that the other chambers remain unaffected. Once closed again, the individual chamber is purged with fresh noble gas, valves opened, and it is returned to the series. Such a multiple plasma chamber configuration would be useful for high throughput applications.

In a still further embodiment, plasma chamber 510 can include doors or flaps at the gas input 540 and gas output 550. Before accessing plasma chamber 510, the doors or flaps can be closed on the inflow and outflow tubes of plasma chamber 510 in order to seal off the rest of the system from the ambient environment. After the next item is loaded in plasma chamber 510, the chamber is purged with fresh gas, and the doors or flaps are then reopened. Using the doors or flaps, only plasma chamber 510 needs to be purged and refilled with the gas, rather than the whole system. Alternatively, fill port 650 and exit port 660 can be located anywhere in the system, including at the plasma chamber 510. Therefore, instead of doors or flaps, fill port 650 and exit port 660 can be used to purge plasma chamber 510. For example, a gas cartridge can be connected to fill port 650 to refill plasma chamber 510. Using this approach, the gas in the remainder of the system (i.e., the gas that is "walled off') would take a substantial amount of time to become sufficiently contaminated as to adversely affect the cold plasma generation process, and thereby require more extensive purging. Using the doors or flaps thereby reduces gas consumption. In a hospital sterilization setting, small gas cartridges can be used rather than large gas cylinders to supply gas to the cold plasma sterilization system.

### Cold Plasma Sterilization Usage Methods

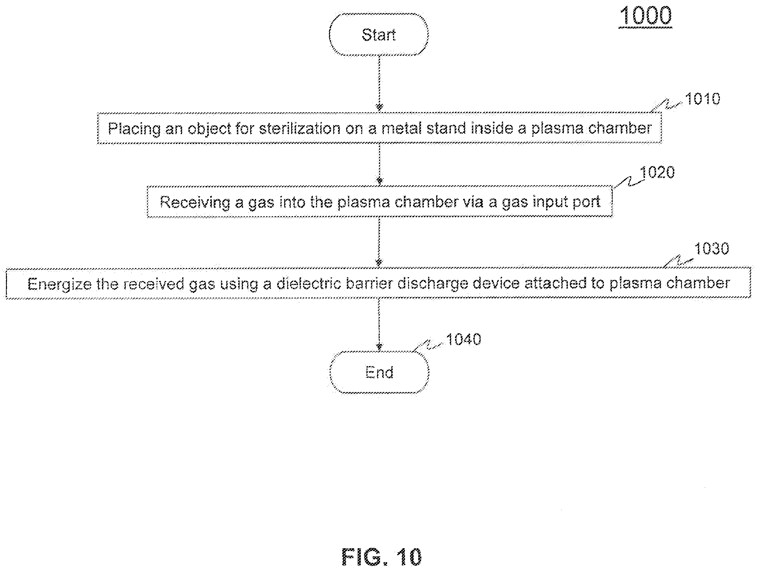

FIG. 10 provides a flowchart of an exemplary method 1000 to generate a cold plasma using a cold plasma treatment device, according to an embodiment of the present invention.

The process begins at step 1010. In step 1010, an object for sterilization is placed on a metal stand inside a plasma chamber, wherein the conductive stand is coupled to ground and configured to accept an object for sterilization, and wherein the plasma chamber includes a gas input port and a gas exit port. In an embodiment, an object 820 is placed on a conductive stand 740 in a plasma chamber 710, having gas input and output ports 750, 760.

In step 1020, gas is received into a plasma chamber. In an embodiment, a gas is received into plasma chamber 710.

In step 1030, the received gas is energized in the plasma chamber to form a cold plasma via one or more dielectric barrier discharge devices attached to the plasma chamber, wherein each of the one or more dielectric barrier discharge devices is formed by a dielectric barrier being sandwiched between an electrode and the interior of the plasma chamber, and wherein each of the electrodes is coupled to a high voltage electric input. In an embodiment, the received gas is energized in plasma chamber 710 using energy from electrodes 720 that is in turn received from electrical input 730. Dielectric barrier 560 is sandwiched between electrode 520 and plasma chamber 510.

At step 1040, method 1000 ends.

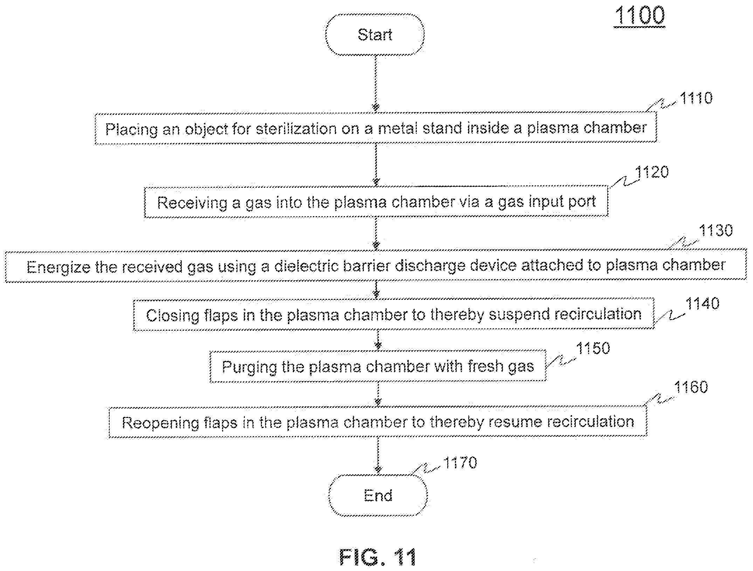

FIG. 11 provides a flowchart of an exemplary method 1100 to generate a cold plasma including recirculation, using a cold plasma treatment device, according to an embodiment of the present invention.

The process begins at step 1110. In step 1110, an object for sterilization is placed on a metal stand inside a plasma chamber, wherein the conductive stand is coupled to ground and configured to accept an object for sterilization, and wherein the plasma chamber includes a gas input port and a gas exit port. In an embodiment, an object 820 is placed on a conductive stand 740 in a plasma chamber 710, having gas input and output ports 750, 760.

In step 1120, gas is received into a plasma chamber. In an embodiment, a gas is received into plasma chamber 710.

In step 1130, the received gas is energized in the plasma chamber to form a cold plasma via one or more dielectric barrier discharge devices attached to the plasma chamber, wherein each of the one or more dielectric barrier discharge devices is formed by a dielectric barrier being sandwiched between an electrode and the interior of the plasma chamber, and wherein each of the electrodes is coupled to a high voltage electric input. In an embodiment, the received gas is energized in plasma chamber 710 using energy from electrodes 720 that is in turn received from electrical input 730. Dielectric barrier 560 is sandwiched between electrode 520 and plasma chamber 510.

In step 1140, flaps in the plasma chamber are closed to seal the plasma chamber. In an embodiment, flaps in plasma chamber 710 are closed to thereby suspend gas recirculation.

In step 1150, the plasma chamber is purged with fresh gas. In an embodiment, plasma chamber 710 is purged with fresh gas with, for example, the use of a gas cartridge to provide the required amount of gas.

In step 1160, the flaps in the plasma chamber are reopened. In an embodiment, flaps in plasma chamber 710 are reopened to thereby resume gas recirculation.

In step 1170, method 1100 ends.

It is to be appreciated that the Detailed Description section, and not the Summary and Abstract sections, is intended to be used to interpret the claims. The Summary and Abstract sections may set forth one or more but not all exemplary embodiments of the present invention as contemplated by the inventor(s), and thus, are not intended to limit the present invention and the appended claims in any way.

The present invention has been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present invention should not be limited by any of the above-described exemplary embodiments, but should be defined only in accordance with the following claims.

## Claims

1. A cold plasma sterilization device (500, 600) comprising:
a plasma chamber (510, 620, 710) comprising a gas input port (540, 750) and a gas output port (550, 760) for throughput of a gas;
a plurality of dielectric barrier discharge devices (400) attached to the plasma chamber (510, 620, 710) and configured to generate a cold plasma within the plasma chamber (510, 620, 710), wherein each of the plurality of dielectric barrier discharge devices (400) is formed by a dielectric barrier (420, 560) being sandwiched between a respective electrode (520, 720, 810, 910) and the interior of the plasma chamber (510, 620, 710), and wherein each of the electrodes (520, 720, 810, 910) is coupled to a high voltage electric input (530, 680); and
a conductive stand (740, 830, 930) disposed within the plasma chamber (510, 620, 710) and configured to accept an object for sterilization, wherein the conductive stand (740, 830, 930) is coupled to ground (440),
wherein the plurality of dielectric barrier discharge devices (400) include a first group and a second group of dielectric barrier discharge devices (400), the first and second group being located on opposing sides of the plasma chamber (510, 620, 710), or wherein the plurality of dielectric barrier discharge devices (400) are distributed evenly with respect to a center of the conductive stand (740, 830, 930).

2. The cold plasma sterilization device (600) of claim 1, further comprising:
a gas recirculation system coupled to the gas input port (540, 750) and the gas output port (550, 760) of the plasma chamber (510, 620, 710), the gas recirculation system comprising a recirculating pump (610) configured to recirculate the gas around the gas recirculation system.

3. The cold plasma sterilization device (600) of claim 2, further comprising:
a fill port (650) for introduction of the gas into the gas recirculation system; and
an exit port (660) for exhaustion of the gas out of the gas recirculation system, or wherein the recirculation pump (610) is a circulation fan.

4. The cold plasma sterilization device (500, 600) of claim 1, wherein the gas comprises a noble gas, or wherein the gas comprises helium.

5. The cold plasma sterilization device (500, 600) of claim 1, wherein the conductive stand (740, 830, 930) comprises two or more conductive stands (740, 830, 930), each configured to accept a respective objective for sterilization, and each coupled to ground (440).

6. The cold plasma sterilization device (500, 600) of claim 1, wherein the plasma chamber (510, 620, 710) further comprises:
a cover (770) having an open position and a closed position, the open position providing external access to the conductive stand (740, 830, 930).

7. A method of creating a cold plasma for sterilisation, the method comprising:
placing (1010, 1110) an object for sterilization on a conductive stand (740, 830, 930) inside a plasma chamber (510, 620, 710), wherein the conductive stand (740, 830, 930) is coupled to ground (440) and configured to accept an object for sterilization, and wherein the plasma chamber (510, 620, 710) includes a gas input port (540, 750) and a gas exit port (550, 760);
receiving (1020, 1120) a gas into the plasma chamber via a gas input port (540, 750), with the gas exiting via a gas output port (550, 760); and
energizing (1030, 1130) the gas in the plasma chamber (510, 620, 710) to generate a cold plasma via a plurality of dielectric barrier discharge devices (400) attached to the plasma chamber (510, 620, 710), wherein each of the plurality of dielectric barrier discharge devices (400) is formed by a dielectric barrier (420, 560) being sandwiched between an electrode (520, 720, 810, 910) and the interior of the plasma chamber (510, 620, 710), and wherein each of the electrodes (520, 720, 810, 910) is coupled to a high voltage electric input (530, 680), and
wherein the energizing (1030, 1130) includes using a first group and a second group of dielectric barrier discharge devices (400), the first and second group being located on opposing sides of the plasma chamber (510, 620, 710), or wherein the energizing (1030, 1130) includes using a plurality of dielectric barrier discharge devices (400) distributed evenly with respect to a center of the conductive stand (740, 830, 930).

8. The method of claim 7, further comprising:
recirculating, by a recirculating pump (610), the gas around a gas recirculation system, wherein the gas recirculation system is coupled to the gas input port (540, 750) and the gas output port (550, 760) of the plasma chamber (510, 620, 710).

9. The method of claim 8, further comprising:
introducing gas into the gas recirculation system via a fill port (650); and
exhausting the gas out of the gas recirculation system via an exit port (660), or wherein the recirculating by a recirculating pump (610) includes recirculating by a circulation fan.

10. The method of claim 7, wherein the gas comprises a noble gas, or wherein the gas comprises helium.

11. The method of claim 7, wherein the placing (1010, 1110) an object for sterilization on a conductive stand (740, 830, 930) inside a plasma chamber (510, 620, 710) includes placing two or more objects for sterilization on respective conductive stands (740, 830, 930) inside the plasma chamber (510, 620, 710).

12. The method of claim 7, wherein the placing (1010, 1110) an object for sterilization on a conductive stand (740, 830, 930) inside a plasma chamber (510, 620, 710) includes accessing the conductive stand (740, 830, 930) via a cover (770) associated with the plasma chamber (510, 620, 710), the cover (770) having an open position and a closed position, with the open position providing the access to the conductive stand (740, 830, 930).

13. The method of claim 8, further comprising:
closing (1140) flaps in the plasma chamber (510, 620, 710) to thereby seal the plasma chamber (510, 620, 710) and suspend gas recirculation;
purging (1150) the plasma chamber (510, 620, 710) with fresh gas; and
opening (1160) the flaps in the plasma chamber to thereby resume gas circulation.

## Patentansprüche

1. Kaltplasmasterilisationsvorrichtung (500, 600), Folgendes beinhaltend:
eine Plasmakammer (510, 620, 710), beinhaltend eine Gaseinlassöffnung (540, 750) und eine Gasauslassöffnung (550, 760) zum Durchfluss eines Gases;
eine Vielzahl von dielektrischen Sperrschichtentladungsvorrichtungen (400), welche an der Plasmakammer (510, 620, 710) befestigt und konfiguriert ist, um ein Kaltplasma innerhalb der Plasmakammer (510, 620, 710) zu erzeugen, wobei eine jede der Vielzahl von dielektrischen Sperrschichtentladungsvorrichtungen (400) durch eine dielektrische Sperrschicht (420, 560) gebildet ist, welche sandwichartig zwischen einer jeweiligen Elektrode (520, 720, 810, 910) und dem Inneren der Plasmakammer (510, 620, 710) angeordnet ist, und wobei jede der Elektroden (520, 720, 810, 910) mit einem elektrischen Hochspannungseingang (530, 680) gekoppelt ist; und
einen leitfähige Ständer (740, 830, 930), welcher innerhalb der Plasmakammer (510, 620, 710) angeordnet und konfiguriert ist, um ein Objekt zur Sterilisation aufzunehmen, und wobei der leitfähige Ständer (740, 830, 930) mit der Erde gekoppelt ist (440),
wobei die Vielzahl von dielektrischen Sperrschichtentladungsvorrichtungen (400) eine erste Gruppe und eine zweite Gruppe von dielektrischen Sperrschichtentladungsvorrichtungen (400) beinhaltet, wobei die erste und die zweite Gruppe an gegenüberliegenden Seiten der Plasmakammer (510, 620, 710) befindlich sind, oder wobei die Vielzahl von dielektrischen Sperrschichtentladungsvorrichtungen (400) gleichmäßig in Bezug auf eine Mitte des leitfähigen Ständers (740, 830, 930) verteilt ist.

2. Kaltplasmasterilisationsvorrichtung (600) nach Anspruch 1, zudem Folgendes beinhaltend:
ein Gasumwälzungssystem, welches mit der Gaseinlassöffnung (540, 750) und der Gasauslassöffnung (550, 760) der Plasmakammer (510, 620, 710) gekoppelt ist, wobei das Gasumwälzungssystem eine Umwälzpumpe (610) beinhaltet, welche konfiguriert ist, um das Gas um das Gasumwälzungssystem umzuwälzen.

3. Kaltplasmasterilisationsvorrichtung (600) nach Anspruch 2, zudem Folgendes beinhaltend:
eine Füllöffnung (650) zum Einleiten des Gases in das Gasumwälzungssystem; und
eine Austrittsöffnung (660) zum Ablassen des Gases aus dem Gasumwälzungssystem, oder wobei die Umwälzpumpe (610) ein Umlauflüfter ist.

4. Kaltplasmasterilisationsvorrichtung (500, 600) nach Anspruch 1, bei welcher das Gas ein Edelgas beinhaltet, oder bei welchem das Gas Helium beinhaltet.

5. Kaltplasmasterilisationsvorrichtung (500, 600) nach Anspruch 1, bei welcher der leitfähige Ständer (740, 830, 930) zwei oder mehr leitfähige Ständer (740, 830, 930) beinhaltet, welche jeweils konfiguriert sind, ein jeweiliges Objekt zur Sterilisation aufzunehmen, und jeweils mit der Erde (440) gekoppelt sind.

6. Kaltplasmasterilisationsvorrichtung (500, 600) nach Anspruch 1, bei welcher die Plasmakammer (510, 620, 710) zudem Folgendes beinhaltet:
eine Abdeckung (770), welche eine geöffnete Position und eine geschlossene Position besitzt, wobei die geöffnete Position externen Zugang zum leitfähigen Ständer (740, 830, 930) bietet.

7. Verfahren zum Erzeugen eines Kaltplasmas zur Sterilisation, wobei das Verfahren Folgendes beinhaltet:
Platzieren (1010, 1110) eines Objekts zur Sterilisation auf einem leitfähigen Ständer (740, 830, 930) innerhalb einer Plasmakammer (510, 620, 710), wobei der leitfähige Ständer (740, 830, 930) mit der Erde (440) gekoppelt und konfiguriert ist, um ein Objekt zur Sterilisation aufzunehmen, und wobei die Plasmakammer (510, 620, 710) eine Gaseinlassöffnung (540, 750) und eine Gasauslassöffnung (550, 760) beinhaltet;
Aufnehmen (1020, 1120) eines Gases in die Plasmakammer über eine Gaseinlassöffnung (540, 750), wobei das Gas über eine Gasauslassöffnung (550, 760) austritt; und
Beaufschlagen mit Energie (1030, 1130) des Gases in der Plasmakammer (510, 620, 710), um ein Kaltplasma über eine Vielzahl von dielektrischen Sperrschichtentladungsvorrichtungen (400) zu erzeugen, welche an der Plasmakammer (510, 620, 710) befestigt sind, zu erzeugen, wobei eine jede der Vielzahl von dielektrischen Sperrschichtentladungsvorrichtungen (400) durch eine dielektrische Sperrschicht (420, 560) gebildet ist, welche sandwichartig zwischen einer Elektrode (520, 720, 810, 910) und dem Inneren der Plasmakammer (510, 620, 710) angeordnet ist, und wobei jede der Elektroden (520, 720, 810, 910) mit einem elektrischen Hochspannungseingang (530, 680) gekoppelt ist, und
wobei das Beaufschlagen mit Energie (1030, 1130) den Gebrauch einer ersten Gruppe und einer zweiten Gruppe von dielektrischen Sperrschichtentladungsvorrichtungen (400) beinhaltet, wobei die erste und die zweite Gruppe an gegenüberliegenden Seiten der Plasmakammer (510, 620, 710) befindlich sind, oder wobei das Beaufschlagen mit Energie (1030, 1130) den Gebrauch einer Vielzahl von dielektrischen Sperrschichtentladungsvorrichtungen (400) beinhaltet, welche gleichmäßig in Bezug auf eine Mitte des leitfähigen Ständers (740, 830, 930) verteilt ist.

8. Verfahren nach Anspruch 7, zudem beinhaltend:
Umwälzen, durch eine Umwälzpumpe (610), des Gases um ein Gasumwälzungssystem, wobei das Gasumwälzungssystem mit der Gaseinlassöffnung (540, 750) und der Gasauslassöffnung (550, 760) der Plasmakammer (510, 620, 710) gekoppelt ist.

9. Verfahren nach Anspruch 8, zudem beinhaltend:
Einleiten von Gas in das Gasumwälzungssystem über eine Füllöffnung (650); und
Ablassen des Gases aus dem Gasumwälzungssystem über eine Austrittsöffnung (660), oder wobei das Umwälzen durch eine Umwälzpumpe (610) Umwälzen durch einen Umlauflüfter beinhaltet.

10. Verfahren nach Anspruch 7, bei welcher das Gas ein Edelgas beinhaltet, oder bei welchem das Gas Helium beinhaltet.

11. Verfahren nach Anspruch 7, bei welchem das Platzieren (1010, 1110) eines Objekts zur Sterilisation auf einem leitfähigen Ständer (740, 830, 930) innerhalb einer Plasmakammer (510, 620, 710) Platzieren von zwei oder mehr Objekten zur Sterilisierung auf jeweiligen leitfähigen Ständern (740, 830, 930) innerhalb der Plasmakammer (510, 620, 710) beinhaltet.

12. Verfahren nach Anspruch 7, bei welchem das Platzieren (1010, 1110) eines Objekts zur Sterilisation auf einem leitfähigen Ständer (740, 830, 930) innerhalb einer Plasmakammer (510, 620, 710) Zugang zum leitfähigen Ständer (740, 830, 930) über eine Abdeckung (770) beinhaltet, welche mit der Plasmakammer (510, 620, 710) assoziiert ist, wobei die Abdeckung (770) eine geöffnete Position und eine geschlossene Position besitzt, wobei die geöffnete Position den Zugang zum leitfähigen Ständer (740, 830, 930) bietet.

13. Verfahren nach Anspruch 8, zudem beinhaltend:
Schließen (1140) von Klappen in der Plasmakammer (510, 620, 710), um hierdurch die Plasmakammer (510, 620, 710) dicht zu verschließen und Gasumwälzung aufzuheben;
Spülen (1150) der Plasmakammer (510, 620, 710) mit frischem Gas; und
Öffnen (1160) der Klappen in der Plasmakammer, um die Gasumwälzung wieder aufzunehmen.

## Revendications

1. Dispositif de stérilisation au plasma à froid (500, 600) comprenant :
une chambre à plasma (510, 620, 710) comprenant un orifice d'entrée de gaz (540, 750) et un orifice de sortie de gaz (550, 760) pour le débit d'un gaz ;
une pluralité de dispositifs de décharge à barrière diélectrique (400) fixés à la chambre à plasma (510, 620, 710) et configurés pour générer un plasma froid dans la chambre à plasma (510, 620, 710), dans lequel chacun de la pluralité de dispositifs de décharge à barrière diélectrique (400) est formé d'une barrière diélectrique (420, 560) prise en sandwich entre une électrode respective (520, 720, 810, 910) et l'intérieur de la chambre à plasma (510, 620, 710) et dans lequel chacune des électrodes (520, 720, 810, 910) est raccordée à une entrée électrique haute tension (530, 680) ; et
un support conducteur (740, 830, 930) disposé dans la chambre à plasma (510, 620, 710), et configuré pour accepter un objet pour stérilisation, dans lequel le support conducteur (740, 830, 930) est raccordé à la terre (440),
dans lequel la pluralité de dispositifs de décharge à barrière diélectrique (400) incluent un premier groupe et un second groupe de dispositifs de décharge à barrière diélectrique (400), le premier et le second groupes étant situés sur des côtés opposés de la chambre à plasma (510, 620, 710), ou dans lequel la pluralité de dispositifs de décharge à barrière diélectrique (400) sont répartis uniformément relativement à un centre du support conducteur (740, 830, 930).

2. Dispositif de stérilisation au plasma à froid (600) selon la revendication 1, comprenant en outre :
un système de recirculation de gaz raccordé à l'orifice d'entrée de gaz (540, 750) et à l'orifice de sortie de gaz (550, 760) de la chambre à plasma (510, 620, 710), le système de recirculation de gaz comprenant une pompe de recirculation (610) configurée pour faire recirculer le gaz autour du système de recirculation de gaz.

3. Dispositif de stérilisation au plasma à froid (600) selon la revendication 2, comprenant en outre :
un orifice de remplissage (650) pour l'introduction du gaz dans le système de recirculation de gaz ; et
un orifice de sortie (660) pour l'échappement des gaz hors du système de recirculation de gaz, ou dans lequel la pompe de recirculation (610) est un ventilateur de circulation.

4. Dispositif de stérilisation au plasma à froid (500, 600) selon la revendication 1, dans lequel le gaz comprend un gaz noble, ou dans lequel le gaz comprend de l'hélium.

5. Dispositif de stérilisation au plasma à froid (500, 600) selon la revendication 1, dans lequel le support conducteur (740, 830, 930) comprend deux supports conducteurs ou plus (740, 830, 930), chacun étant configuré pour accepter un objectif respectif pour la stérilisation, et chacun étant raccordé à la terre (440).

6. Dispositif de stérilisation au plasma à froid (500, 600) selon la revendication 1, dans lequel la chambre à plasma (510, 620, 710) comprend en outre :
un capot (770) présentant une position ouverte et une position fermée, la position ouverte fournissant un accès extérieur au support conducteur (740, 830, 930).

7. Procédé de création d'un plasma froid pour stérilisation, le procédé comprenant :
le placement (1010, 1110) d'un objet pour stérilisation sur un support conducteur (740, 830, 930) à l'intérieur d'une chambre à plasma (510, 620, 710), dans lequel le support conducteur (740, 830, 930) est raccordé à la terre (440) et configuré pour accepter un objet pour stérilisation, et dans lequel la chambre à plasma (510, 620, 710) inclut un orifice d'entrée de gaz (540, 750) et un orifice de sortie de gaz (550, 760) ;
la réception (1020, 1120) d'un gaz dans la chambre à plasma via un orifice d'entrée de gaz (540, 750) avec le gaz sortant par le biais d'un orifice de sortie de gaz (550, 760) ; et
l'excitation (1030, 1130) du gaz dans la chambre à plasma (510, 620, 710), afin de générer un plasma froid via une pluralité de dispositifs de décharge à barrière diélectrique (400) fixés à la chambre à plasma (510, 620, 710), dans lequel chacun de la pluralité de dispositifs de décharge à barrière diélectrique (400) est formé d'une barrière diélectrique (420, 560) prise en sandwich entre une électrode (520, 720, 810, 910) et l'intérieur de la chambre à plasma (510, 620, 710), et dans lequel chacune des électrodes (520, 720, 810, 910) est raccordée à une entrée électrique haute tension (530, 680) et
dans lequel l'excitation (1030, 1130) inclut l'utilisation d'un premier groupe et d'un second groupe de dispositifs de décharge à barrière diélectrique (400), le premier et le second groupes étant situés sur des côtés opposés de la chambre à plasma (510, 620, 710), ou dans lequel l'excitation (1030, 1130) inclut l'utilisation d'une pluralité de dispositifs de décharge à barrière diélectrique (400) répartis uniformément relativement à un centre d'un support conducteur (740, 830, 930).

8. Procédé selon la revendication 7, comprenant en outre :
la recirculation, par une pompe de recirculation (610), du gaz autour d'un système de recirculation de gaz, dans lequel le système de recirculation de gaz est raccordé à l'orifice d'entrée de gaz (540, 750) et à l'orifice de sortie de gaz (550, 760) de la chambre à plasma (510, 620, 710).

9. Procédé selon la revendication 8, comprenant en outre :
l'introduction d'un gaz dans le système de recirculation de gaz via un orifice de remplissage (650) ; et
l'échappement du gaz hors du système de recirculation de gaz via un orifice de sortie (660) ou dans lequel la recirculation par une pompe de recirculation (610) inclut la recirculation par un ventilateur de circulation.

10. Procédé selon la revendication 7, dans lequel le gaz comprend un gaz noble, ou dans lequel le gaz comprend de l'hélium.

11. Procédé selon la revendication 7, dans lequel le placement (1010, 1110) d'un objet pour stérilisation sur un support conducteur (740, 830, 930) à l'intérieur d'une chambre à plasma (510, 620, 710), inclut le placement de deux objets ou plus pour stérilisation sur des supports conducteurs respectifs (740, 830, 930) à l'intérieur de la chambre à plasma (510, 620, 710).

12. Procédé selon la revendication 7, dans lequel le placement (1010, 1110) d'un objet pour stérilisation sur un support conducteur (740, 830, 930) à l'intérieur d'une chambre à plasma (510, 620, 710), inclut l'accès au support conducteur (740, 830, 930) via un capot (770) associé à la chambre à plasma (510, 620, 710), le capot (770) présentant une position ouverte et une position fermée, la position ouverte permettant un accès au support conducteur (740, 830, 930).

13. Procédé selon la revendication 8, comprenant en outre :
la fermeture (1140) des volets dans la chambre à plasma (510, 620, 710) afin de sceller ainsi la chambre à plasma (510, 620, 710) et de suspendre la recirculation de gaz ;
la purge (1150) de la chambre à plasma (510, 620, 710) avec du gaz frais ; et
l'ouverture (1160) des volets dans la chambre à plasma afin de reprendre ainsi la circulation de gaz.
